(19) Europäisches Patentamt
European Patent Office
Office européen des brevets

(11) Veröffentlichungsnummer: **0 237 467 B1**

(12) **EUROPÄISCHE PATENTSCHRIFT**

(45) Veröffentlichungstag der Patentschrift: **28.10.92**

(51) Int. Cl.$^5$: **C07D 471/04**, A61K 31/44, A61K 31/50, A61K 31/505, //(C07D471/04,221:00,209:00)

(21) Anmeldenummer: **87730022.8**

(22) Anmeldetag: **06.03.87**

Die Akte enthält technische Angaben, die nach dem Eingang der Anmeldung eingereicht wurden und die nicht in dieser Patentschrift enthalten sind.

(54) **Hetaryl-oxy-beta-carbolinderivate, ihre Herstellung und Verwendung als Arzneimittel.**

(30) Priorität: **08.03.86 DE 3608089**

(43) Veröffentlichungstag der Anmeldung: **16.09.87 Patentblatt 87/38**

(45) Bekanntmachung des Hinweises auf die Patenterteilung: **28.10.92 Patentblatt 92/44**

(84) Benannte Vertragsstaaten: **AT BE CH DE ES FR GB GR IT LI LU NL SE**

(56) Entgegenhaltungen:
**EP-A- 0 030 254**
**EP-A- 0 054 507**
**EP-A- 0 130 140**
**EP-A- 0 161 574**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Biere, Helmut, Dr.**
**Zeltinger Strasse 15**
**W-1000 Berlin 28(DE)**

Erfinder: **Huth, Andreas, Dr.**
**Kirchweg 55**
**W-1000 Berlin 38(DE)**
Erfinder: **Rahtz, Dieter, Dr.**
**Krottnaurerstrasse 24a**
**W-1000 Berlin 38(DE)**
Erfinder: **Schmiechen, Ralph, Dr.**
**Bayernring 27**
**W-1000 Berlin 42(DE)**
Erfinder: **Seidelmann, Dieter, Dr.**
**Stierstrasse 14**
**W-1000 Berlin 41(DE)**
Erfinder: **Stephens, David Norman, Dr.**
**Hildegardstrasse 16a**
**W-1000 Berlin 31(DE)**
Erfinder: **Engelstoft, Mogens, Dr.**
**Mosegard Park 121**
**DK-3500 Vaerlose(DK)**
Erfinder: **Hansen, John Bondo, Dr.**
**Havretoften 10**
**DK-2800 Lyngby(DK)**

EP 0 237 467 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Hetaryloxy-β-carbolinderivate, ihre Herstellung und ihre Verwendung als Arzneimittel.

Die erfindungsgemäßen Verbindungen besitzen wertvolle pharmakologische Eigenschaften. Sie beeinflussen insbesondere das Zentralnervensystem und eignen sich somit als Psychopharmaka.

Die erfindungsgemäßen Hetaryloxy-β-carbolinderivate haben die allgemeine Formel I

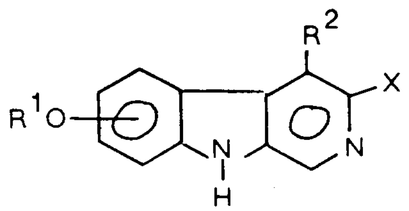

(I),

worin

$R^1$ ein 6-Ring-Hetarylrest mit 1-2 Stickstoffatomen oder ein 5-Ring-Hetarylrest mit 1-2 Sauerstoff-, Schwefel- und/oder Stickstoffatomen, der gegebenenfalls ein- oder mehrfach substituiert ist mit Halogenen, Nitro, Amino, Cyano, $C_{1-6}$-Alkyl oder $C_{1-6}$-Alkoxy-carbonyl.

$R^2$ Wasserstoff, $C_{1-6}$-Alkyl oder $C_{1-6}$-Alkoxyalkyl und

X eine $COOR^3$-Gruppe mit $R^3$ in der Bedeutung von H oder $C_{1-6}$-Alkyl oder

eine $CONR^4R^5$-Gruppe darstellt mit $R^4$ und $R^5$ jeweils in der Bedeutung von Wasserstoff oder $C_{1-3}$-Alkyl, wobei $R^4$ und $R^5$ gemeinsam mit dem Stickstoffatom Morpholin, Piperidin, Thiomorpholin, Piperazin, Pyrrolidin, Imidazolidin, Pyrazolidin, Isothiazolidin bilden können oder

einen Oxadiazolylrest der Formel

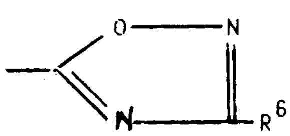

oder

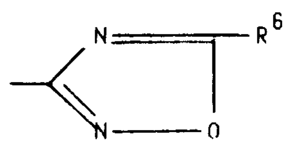

mit $R^6$ in der Bedeutung von Wasserstoff, $C_{1-6}$-Alkyl oder $C_{3-5}$-Cycloalkyl bedeutet.

Der Substituent $OR^1$ kann in 5-, 6-, 7- oder 8-Stellung, vorzugsweise in 5- oder 6-Stellung des β-Carbolins, stehen.

Der Heteroaromat $R^1$ ist beispielsweise 5- oder 6-gliedrig und kann ein- oder mehrfach substituiert sein, wobei der Substituent in jeder beliebigen Position des Heteroaromaten stehen kann.

Als 6-Ring-Heteroaromaten sind bevorzugt stickstoffhaltige Aromaten geeignet, die 1-2 Stickstoffatome enthalten können, wie beispielsweise Pyridin, Pyrimidin, Pyrazin und Pyridazin.

Als 5-Ring-Heteroaromaten sind sauerstoff-, schwefel- und/oder stickstoffhaltige Aromaten geeignet wie beispielsweise Furan, Thiazol, Thiophen, Pyrrol oder Imidazol, die im allgemeinen 1-2 Heteroatome enthalten können.

Als Substituenten des Heteroaromaten kommen beispielsweise Halogene wie Fluor, Chlor oder Brom, Nitro, Amino, Cyano, $C_{1-6}$-Alkyl und $C_{1-6}$-Alkoxy-carbonylgruppen in Betracht.

Unter $C_{1-6}$-Alkyl sind sowohl gerad- als auch verzweigtkettige gesättigte Reste zu verstehen. Beispielsweise seien genannt Methyl, Ethyl, n-Propyl, iso-Propyl, n-Butyl, iso-Butyl, tert. -Butyl, sek. Butyl, Pentyl und Hexyl. Als bevorzugt sind $C_{1-4}$ Alkylreste anzusehen.

Als bevorzugt für die Reste $R^4$ und $R^5$ seien $C_{1-3}$-Alkyle genannt. Bilden $R^4$ und $R^5$ gemeinsam mit dem Stickstoffatom einen Heterocyclus, so ist dieser gesättigt und 5-6 gliedrlg und kann ein weiteres Heteroatom wie Schwefel, Stickstoff oder Sauerstoff enthalten wie beispielsweise Morpholin, Piperidin, Thiomorpholin, Piperazin, Pyrrolidin, Imidazolidin, Pyrazolidin, Isothiazolidin.

Der Cycloalkylrest $R^6$ kann 3-7 Kohlenstoffatome enthalten, wobei als bevorzugt Reste mit 3-5 Kohlenstoffatomen genannt seien wie beispielsweise Cyclopropyl, Methylcyclopropyl, Cyclobutyl oder Cyclopentyl.

Es ist bekannt, daß bestimmte Stellen im zentralen Nervensystem von Vertebraten eine hohe spezifische Affinität für die Bindung von 1.4 und 1.5-Benzodiazepinen aufweisen (Squires, R.F. und Braestrup, C., Nature (London) 266 (1977), 734). Diese Stellen werden Benzodiazepin-Rezeptorengenannt.

Die für die pharmakologischen Eigenschaften der erfindungsgemäßen Verbindungen wichtige Rezeptoraffinität wurde durch Untersuchung des Verdrängungsvermögens von radioaktiv-markiertem Flunitrazepam von Benzodiazepin-Rezeptoren bestimmt.

Die Verdrängungsaktivität der erfindungsgemäßen Verbindungen wird als $IC_{50}$- und $ED_{50}$-Wert angegeben. Der $IC_{50}$-Wert gibt die Konzentration an, die eine 50 %ige Verdrängung der spezifischen Bindung von $H^3$-Flunitrazepam (1,0 nM, 0 °C) in Proben mit einem Gesamtvolumen von 0,55 ml einer Suspension von Hirnmembranen, z.B. von Ratten bewirkt.

Der Verdrängungstest wird wie folgt ausgeführt:

0,5 ml einer Suspension von unbehandelten Rattenvorderhirnen in 25 mM $KH_2PO_4$, pH = 7,1 (5-10 mg Gewebe/Probe) wird für 40 bis 60 Minuten bei 0 °C zusammen mit $^3H$-Flunitrazepam (spezifische Aktivität 87 Ci/mmol, 1nM) inkubiert. Nach Inkubation wird die Suspension durch eine Glasfritte filtriert, der Rückstand zweimal mit kalter Pufferlösung gewaschen und die Radioaktivität im Scintillationszähler gemessen.

Der Versuch wird dann wiederholt, jedoch so, daß vor der Zugabe des radioaktiv-markiertem Benzodiazepins eine bestimmte Menge oder eine überschüssige Menge der Verbindung, deren Verdrängungsaktivität zu bestimmen ist, zugesetzt wird. Auf Grundlage der erhaltenen Werte kann dann der $IC_{50}$-Wert berechnet werden.

Der $ED_{50}$-Wert stellt die Dosis einer Versuchssubstanz dar, die eine Reduktion der spezifischen Bindung des Flunitrazepams an dem Benzodiazepin-Rezeptor in einem lebenden Gehirn auf 50 % des Kontrollwertes bewirkt.

Der in-vivo Test wird wie folgt ausgeführt:

Gruppen von Mäusen wird die Versuchssubstanz bei unterschiedlichen Dosen und normalerweise intraperitoneal injiziert. Nach 15 Minuten wird den Mäusen das $^3H$-Flunitrazepam intravenös verabreicht. Nach weiteren 20 Minuten werden die Mäuse getötet, ihr Vorderhirn entfernt und die an die Hirnmembranen spezifisch gebundene Radioaktivität durch Scintillationszählung gemessen. Der $ED_{50}$-Wert wird aus den Dosis/Wirkungs-Kurven bestimmt.

Die neuen Verbindungen der allgemeinen Formel I besitzen wertvolle pharmakologische Eigenschaften. Insbesondere wirken sie auf da Zentralnervensystem und sind somit als Psychopharmaka in der Humanmedizin geeignet.

Aus EP-A-130140, EP-A-54507, EP-A-161574 und EP-A-30254 sind β-Carboline bekannt, die mit einem Aryloxy-Rest substituiert sind oder einen Hetarylrest enthalten, der direkt an das Carbolingerüst gebunden ist. Diese Verbindungen weisen geringe Affinität an die Benzodiazepin-Rezeptoren auf und zeigen geringe anxiolytische Wirksamkeit.

Die erfindungsgemäßen Verbindungen zeichnen sich insbesondere durch anxiolytische und antikonvulsive Wirksamkeit aus. Zur Untersuchung der anxiolytischen Wirkung wurden die Verbindungen in 4-Plattentest nach der Methode von Boissier et al Eur. J. Pharmacol. 4, 145-150 (1968) getestet. In der Tabelle ist die minimale niedrigste Dosis (MED) angegeben, die die lokomotorische Aktivität der gestraften Mäuse nach i.p. Behandlung erhöht.

TABELLE

| 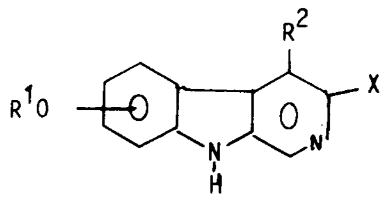 | | | Hemmung der $H^3$-Flunitrazepam-Bindung | | anxiolytische Wirkung |
|---|---|---|---|---|---|
| $R^1$ | $R^2$ | X | $IC_{50}$ ng/ml in vitro | $ED_{50}$ mg/kg in vivo | MED mg/kg i.p. |
| 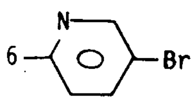 | $CH_2OCH_3$ | COO-i-prop | 0,75 | 2,7 | 3,13 |
| 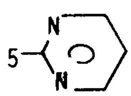 | H | 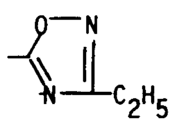 | 0,28 | 2,8 | 3,13 |

Die Verbindungen der allgemeinen Formel 1 können besonders zur Behandlung von Angst begleitet von Depressionen, Epilepsie, Schlafstörungen, Spastizitäten und Muskelrelaxation während der Anästhesie eingesetzt werden. Die erfindungsgemäßen Verbindungen zeigen auch amnestische bzw. gedächtnisfördernde Eigenschaften.

Die erfindungsgemäßen Verbindungen können zur Formulierung von pharmazeutischen Präparationen, zum Beispiel für die orale und parentale Anwendung nach an sich bekannten Methoden der Galenik verwendet werden.

Als Hilfsstoffe zur Formulierung von pharmazeutischen Präparationen sind solche physiologisch verträglichen organischen und anorganischen Trägersubstanzen für die enterale und parenterale Anwendung geeignet, die gegenüber den erfindungsgemäßen Verbindungen inert sind.

Als Trägersubstanzen seien beispielsweise genannt: Wasser, Salzlösungen, Alkohole, Polyäthylenglykole, polyhydroxyethoxyliertes Rizinusöl, Gelatine, Lactose, Amylose, Magnesiumstearat, Talkum, Kieselsäure, Fettsäuremono- und diglyceride, Pentaerythritolfettsäureester, Hydroxymethylcellulose und Polyvinylpyrrolidon.

Die pharmazeutischen Präparationen können sterilisiert und/oder mit Hilfsstoffen, wie Schmiermitteln, Konservierungsstoffen, Stabilisatoren, Netzmitteln, Emulgatoren, Puffermitteln und Farbstoffen, versetzt werden.

Für die parenterale Anwendung sind insbesondere Injektionslösungen oder Suspensionen, insbesondere wässrige Lösungen der aktiven Verbindungen in polyhydroxyethoxyliertem Rizinusöl, geeignet.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt wird.

Die erfindungsgemäßen Verbindungen werden in einer Dosiseinheit von 0,05 bis 100 mg aktiver Substanz in einem physiologisch verträglichen Träger eingebracht.

Die erfindungsgemäßen Verbindungen werden in einer Dosis von 0,1 bis 300 mg/Tag, vorzugsweise 1 bis 30 mg/Tag, angewendet.

Die Herstellung der erfindungsgemäßen Verbindungen erfolgt nach an sich bekannten Methoden.

Beispielsweise erfolgt die Herstellung der Verbindungen der allgemeinen Formel I dadurch, daß man

a) ein Indol der allgemeinen Formel II

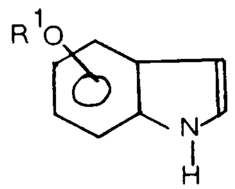

(II),

worin $R^1$ die in Formel I angegebene Bedeutung hat, mit einem Azabutadien der Formel III

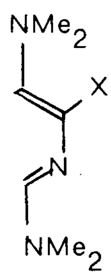

(III),

worin X eine $COOR^3$-Gruppe mit $R^3$ in der Bedeutung von $C_{1-6}$-Alkyl oder einen Oxadiazolylrest der Formel

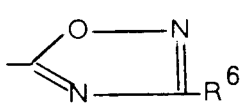

oder

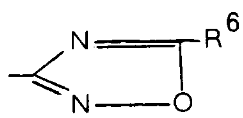

mit $R^6$ in der oben genannten Bedeutung darstellt, in Gegenwart von Säuren umsetzt oder

b) ein $\beta$-Carbolinderivat der allgemeinen Formel IV

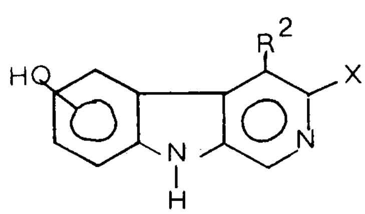

(IV),

worin X und $R^2$ die oben angegebene Bedeutung haben, mit Halogen $R^1$ veräthert, wobei $R^1$ die oben angegebene Bedeutung hat oder

c) eine Verbindung der Formel V

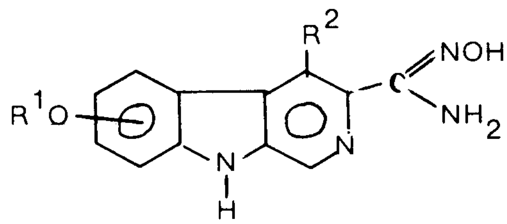

(V),

worin $R^1$ und $R^2$ die oben angegebene Bedeutung haben mit einer Verbindung der Formel $(R^6CO)_2O$ mit $R^6$ in der oben genannten Bedeutung umsetzt zu einer Verbindung der allgemeinen Formel I mit X in der Bedeutung von

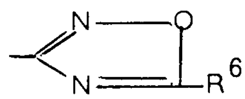

mit $R^6$ in der oben genannten Bedeutung und anschließend gegebenenfalls die nach Verfahren a), b) oder c) hergestellten Verbindungen

α) mit $R^1$ in der Bedeutung von $O_2N$-Hetaryl reduziert zu $H_2N$-Hetaryl-Verbindungen und diese gewünschtenfalls anschließend in N≡C-Hetaryl-Verbindungen überführt und

β) mit $R^1$ in der Bedeutung von Halogenhetaryl enthalogeniert und

γ) mit X in der Bedeutung von $COOR^3$, worin $R^3$ $C_{1-6}$-Alkyl bedeutet, umestert oder verseift und die so erhaltenen Verbindungen mit $R^3$ in der Bedeutung von Wasserstoff gewünschtenfalls amidiert zu Verbindungen mit X in der Bedeutung von $CONR^4R^5$, wobei $R^4$ und $R^5$ die oben genannte Bedeutung haben oder mit einem Amidoxim der Formel $R^6$-$CNH_2$(=NOH),worin $R^6$ die oben angegebene Bedeutung hat, umsetzt zu Verbindungen mit X in der Bedeutung von

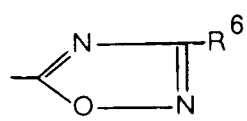

worin $R^6$ die oben angegebene Bedeutung hat.

Nach Verfahren a) erfolgt die Umsetzung des Indolderivats der allgemeinen Formel II mit dem Azadien in Gegenwart von Säuren bei Temperaturen zwischen 50 und 200 °C. Die Umsetzung wird beispielsweise so ausgeführt, daß das Indolderivat und das Azabutadien der Formel III in einer aliphatischen Carbonsäure wie Ameisensäure, Essigsäure, Propionsäure oder Trifluoressigsäure, oder in einem anorganischen Milieu, wie in Phosphorsäure oder Polyphosphorsäure erhitzt werden. Es können auch inerte organische Lösungsmittel, wie zum Beispiel Toluol, Ethylacetat, Dioxan, Dimethoxyethan, Acetonitril oder Dichlormethan zugesetzt werden.

Die Umsetzung kann aber auch in Gegenwart von katalytischen Mengen einer Mineralsäure wie Schwefelsäure, Salzsäure, Perchlorsäure etc. in einem der zuvor genannten inerten Lösungsmittel ausgeführt werden und ist im allgemeinen nach 2 bis 10 Stunden beendet.

Die Verätherung der β-Carbolinderivate der allgemeinen Formel IV nach Verfahren b) erfolgt beispielsweise, indem man eine reaktionsfähige Hetarylverbindung in einem polaren Lösungsmittel wie beispielsweise Dimethylsulfoxid, Dimethylformamid, Acetonitril oder Ethanol in Gegenwart einer Base bei Temperaturen bis zum Siedepunkt des Lösungsmittels umsetzt. Als reaktionsfähige Hetarylverbindungen sind insbesondere die Halogenide wie Chlorid, Bromid oder Jodid sowie Mesylat oder Tosylat geeignet.

Als Basen kommen Alkaliverbindungen wie beispielsweise Natrium- oder Kaliumhydroxid, Natrium- oder Kaliumcarbonat in Frage, gegebenenfalls auch in Gegenwart von Phasentransferkatalysatoren wie beispielsweise Kronenäther oder Aliquat 336.

Zweckmäßigerweise wird unter Inertgasatmosphäre, beispielsweise unter Stickstoff oder Argon, gearbeitet.

Die Reduktion der Nitrogruppe zur Aminogruppe kann beispielsweise katalytisch in polaren Lösungsmit-

teln bei Raumtemperatur unter $H_2$-Druck oder Normaldruck vorgenommen werden.

Vorzugsweise wird als Katalysator Palladium auf einem Träger wie Kohle oder Platin in feinverteilter Form verwendet.

Als polare Lösungsmittel sind für die Reduktion geeignet: beispielsweise Alkohole oder Äther wie Methanol, Ethanol, Diethylether, Tetrahydrofuran oder deren Mischungen.

Die Einführung der Cyanogruppe erfolgt beispielsweise nach der Sandmeyer-Reaktion, indem man die aus den Aminoverbindungen mit Nitriten intermediär gebildeten Diazoniumsalze mit Alkalicyaniden in Gegenwart von Cu-I-cyanid umsetzt.

Die katalytische Dehalogenierung wird beispielsweise mit Palladium auf Kohle (10 %) unter Zusatz von organischen Basen wie beispielsweise Triethylamin in Alkohol durchgeführt.

Um Umesterungen zu vermeiden, nimmt man zweckmäßigerweise den Alkohol der Esterkomponente als Lösungsmittel.

Wird eine Umesterung gewünscht, so kann man zum Beispiel mit dem entsprechenden Alkohol oder Alkalialkoholat umsetzen, gegebenenfalls kann man Titantetra-isopropylat als Katalysator im wasserfreien Alkohol zufügen. Üblicherweise wird die Umesterung bei Temperaturen von 60 bis 120 °C durchgeführt und ist nach ca. 2 bis 6 Stunden beendet.

Die Einführung der tert.-Butylestergruppe erfolgt beispielsweise durch Umsetzung der Carbonsäure mit tert.-Butoxy-bis-dimethyl-aminomethan. Im allgemeinen wird die Reaktion unter Inertgasatmosphäre wie Argon oder Stickstoff und unter Feuchtigkeitsausschluß bei erhöhter Temperatur durchgeführt.

Die Verseifung der Estergruppe kann sauer oder alkalisch erfolgen; vorzugsweise wird alkalisch verseift, indem der Ester mit verdünnter wässriger Alkalilauge wie Kalium-oder Natriumhydroxid, in einem protischen Lösungsmittel, wie zum Beispiel Methanol, Ethanol oder Ethylenglykol, auf Temperaturen bis zur Rückflußtemperatur des Reaktionsgemisches erhitzt wird.

Carbonsäureamide werden beispielsweise erhalten durch Umsetzung mit Aminen aus den entsprechenden Imidazoliden, die intermediär hergestellt werden aus den Carbonsäuren und Carbonyl- oder Thionyldiimidazol. Die Reaktion wird bei Raumtemperatur in dipolaren aprotischen Lösungsmitteln durchgeführt wie beispielsweise Dimethylformamid, Dimethylacetamid u.a..

Zur Einführung des 1,2,4-Oxadiazol-5-yl-Restes wird die β-Carbolincarbonsäure zum Beispiel mit einem Amidoxim der Formel

$R^6$-C(=NOH)$NH_2$,

in einem inerten Lösungsmittel, das über 100 °C siedet und gegenüber dem Reaktanten inert ist, bei der Rückflußtemperatur des Reaktionsgemisches zur Kondensation gebracht. Geeignete Lösungsmittel für die Kondensationsreaktion sind beispielsweise Toluol und Dimethylformamid. Zweckmäßigerweise wird die freie β-Carbolin-3-carbonsäure vor der Kondensationsreaktion in geeigneter Weise aktiviert. Hierzu kann die freie Säure zum Beispiel in das gemischte Anhydrid, in den aktivierten Ester oder in das Chlorid überführt werden.

Gut bewährt hat sich auch eine Aktivierung zum Imidazolid mit Imidazol/Thionylchlorid oder auch Carbonyldiimidazol in einem aprotischen Lösungsmittel wie Dioxan, Tetrahydrofuran, Dimethylformamid oder N-Methylpyrrolidon bei Temperaturen zwischen 0 und 50 °C, vorzugsweise Raumtemperatur.

Die Herstellung von 1,2,4-Oxadiazol-3-yl-β-carbolinderivaten erfolgt beispielsweise aus den β-Carbolin-3-carbonsäuren, indem die wie üblich dargestellten Säureamide mit wasserabspaltenden Mitteln wie zum Beispiel einem Reagenz aus Triphenylphosphin/Brom in Gegenwart von Triethylamin in die entsprechenden Nitrile überführt werden. Diese können anschließend mit Hydroxylamin zu den gewünschten β-Carbolin-3-carboxamidoximen umgesetzt werden. Die so erhaltenen β-Carbolin-3-carboxamidoxime werden bei Raumtemperatur mit dem Säureahydrid $(R^6CO)_2O$ versetzt und anschließend bis zur Siedetemperatur erwärmt. Die Reaktion ist nach ca. 7 Stunden beendet und wird nach dem üblichen Verfahren aufgearbeitet.

Die Herstellung der Ausgangsverbindungen ist beispielsweise aus der EP-A 54 507 und der EP-A 110 813 bekannt oder erfolgt nach bekannten verfahren.

Die nachfolgenden Beispiele sollen das erfindungsgemäße Verfahren erläutern.

Beispiel 1

5-(2-Pyrazinyloxy)-β-carbolin-3-carbonsäure-ethylester

Unter Eiskühlung wird ein Gemisch von 2 ml Eisessig und 0,3 ml Trifluoressigsäure mit 260 mg 1,4-Bis-(dimethyl-amino)-2-azabutadien-3-carbonsäure-ethylester versetzt und 10 Minuten gerührt. Danach werden

211 mg 4-(2-Pyrazinyloxy)-indol zugefügt und die Mischung 24 Stunden bei Raumtemperatur unter Stickstoffatmosphäre gerührt und anschließend 2 Stunden unter Rückfluß (150 - 160 °C Badtemperatur) erhitzt. Nach Abkühlen wird in $K_2CO_3$-Lösung eingegossen, das Kristallisat abgesaugt, mit Wasser nachgewaschen und aus Ethanol umkristallisiert. Man erhält 190 mg (56 %), Schmelzpunkt 264-266 °C (EtoH).

Das Ausgangsmaterial wird auf folgende Weise erhalten:
Eine Lösung von 2,66 g 4-Hydroxyindol in 60 ml DMSO wird mit 1,4 g Kaliumhydroxid (gepulvert) versetzt und unter $N_2$-Atmosphäre 1 Stunde bei Raumtemperatur gerührt. Nach Zugabe von 2,5 g 2-Chlorpyrazin wird 2 Stunden auf 100 °C erhitzt, abgekühlt, in Wasser gegossen und mit Ethylacetat extrahiert. Der Rückstand der organischen Phase wird über Kieselgel gereinigt. Man erhält 2,95 g (70 %) 4-(2-Pyrazinyloxy)-indol, Schmelzpunkt 192-193 °C.

Beispiel 2

5-(5-Nitro-2-pyridyloxy)-β-carbolin-3-carbonsäure-ethylester

Schmelzpunkt 298-300 °C. Analog Beispiel 1 aus 4-(5-Nitro-2-pyridyloxy)-indol.

Das Ausgangsmaterial wird analog Beispiel 1 aus 4-Hydroxyindol und 2-Chlor-5-nitropyridin erhalten. Schmelzpunkt 169-170 °C.

Beispiel 3

5-(2-Pyrimidinyloxy)-β-carbolin-3-carbonsäure-ethylester

Schmelzpunkt 273-275 °C. Analog Beispiel 1 aus 4-(2-Pyrimidinyloxy)-indol.

Das Ausgangsmaterial wird analog Beispiel 1 aus 4-Hydroxyindol und 2-Chlorpyrimidin hergestellt. Schmelzpunkt 233-234 °C (Diisopropylether).

Beispiel 4

3-(3-Ethyl-1,2,4-oxadiazol-5-yl)-5-(2-pyrazinyloxy)-β-carbolin

Unter Eiskühlung wird ein Gemisch aus 4 ml Eisessig und 0,5 ml Trifluoressigsäure mit 340 mg 1,4-Bis-(dimethylamino)-3-(3-ethyl-1,2,4-oxadiazol-5-yl)-2-azabutadien versetzt und 10 Minuten gerührt. Danach werden 211 mg 4-(2-Pyrazinyloxy)-indol zugefügt und die Mischung wird zunächst 24 Stunden bei Raumtemperatur, dann 2 Stunden bei 100 °C und schließlich 6 Stunden unter Rückfluß gehalten. Nach Aufarbeitung mit $K_2CO_3$-Lösung wird das Produkt über Kieselgel chromatographiert. Man erhält 92 mg (25 %). Schmelzpunkt 278-280 °C (EtOH).

Das Azadien wird wie folgt erhalten:

A) 3-Ethyl-5-(phthalimidomethyl)-1,2,4-oxadiazol
Zu einer Lösung von 65,7 g Phthalimidoessigsäure in 500 ml THF (abs.) wird bei 40 °C eine Suspension von 26,0 g Carbonyldiimidazol in 250 ml THF zugefügt. Nach circa 1 Stunde ist keine Gasentwicklung mehr feststellbar. Jetzt wird eine Lösung von 28,2 g Propioamidoxim in 50 ml THF zugesetzt und 24 Stunden bei Raumtemperatur gerührt. Nach Abfiltrieren des Niederschlages wird das Filtrat im Vakuum eingeengt und nach Zusatz von 500 ml trockenem Xylol 6 Stunden am Wasserabscheider unter Rückfluß erhitzt. Die noch heiße Lösung wird vom öligen Rückstand getrennt und im Vakuum eingeengt. Nach Kristallisation aus EtOH erhält man 31,5 g (76,5 % bezogen auf Carbonyldiimidazol) Oxadiazol mit Schmelzpunkt 106-107 °C.

B) 5-Aminomethyl-3-ethyl-1,2,4-oxadiazol
Eine Suspension von 32,2 g Phthalimid in 250 ml Methanol wird bei Raumtemperatur mit 4,5 g (140 mmol) Hydrazin versetzt, wobei sich die Substanz rasch auflöst. Das Reaktionsgemisch wird 3 Stunden unter Rückfluß gekocht, dann der gebildete Niederschlag abgesaugt, mit Methanol nachgewaschen und das Filtrat eingeengt. Nach Aufschlämmen des Rückstandes mit Diethylether wird erneut filtriert, eingeengt und das Öl am Kugelrohr destilliert, Siedepunkt 90-100 °C, O,03 Torr. Ausbeute 14,87 g (91,6 % d.Th.); $n_D^{20}$ 1.4691.

C) Eine Mischung von 11,5 g 5-Aminomethyl-3-ethyl-1,2,4-oxadiazol und 24 ml Dimethylformamiddimethylacetal wird 7 Stunden auf 80 °C erhitzt; dabei werden 10 ml entstandenes Methanol abdestilliert. Nach Zugabe von weiteren 12 ml DMF-acetal wird die Mischung 3 Stunden unter Rückfluß gekocht, dann fraktioniert destilliert. Die bei 155-160 °C und 0,03 Torr übergehende Fraktion 1,4-Bis-(dimethylamino)-3-(3-ethyl-1,2,4-oxadiazol-5-y)-2-aza-1,3-butadien wird in einer Ausbeute von 72 % d.Th. ($n_D^{20}$ 1.5908) erhalten.

Beispiel 5

5-( 5-Chlor-2-pyridyloxy)-3-(3-ethyl-1,2,4-oxadiazol-5-yl)-β-carbolin

Analog Beispiel 4 aus 4-(5-Chlor-2-pyridyloxy)-indol, Schmelzpunkt 259-260 °C (EtOH).

Beispiel 6

5-(2-Pyrimidinyloxy)-3-(3-ethyl-1,2,4-oxadazol-5-yl)-β-carbolin

Analog Beispiel 4 aus 4-(2-Pyrimidinyloxy)-indol, Schmelzpunkt 254-256 °C (EtOH).

Beispiel 7

5-(5-Nitro-2-pyridyloxy)-3-(3-ethyl-1,2,4-oxadiazol-5-yl)-β-carbolin

Analog Beispiel 4 aus 4-(5-Nitro-2-pyridyloxy)-indol

Beispiel 8

4-Methoxymethyl-5-(2-pyrazinyloxy)-β-carbolin-3-carbonsäure-ethylester

Eine Lösung von 300 mg 5-Hydroxy-4-methoxymethyl-β-carbolin-3-carbonsäure-ethylester in 3 ml trockenem Dimethylsulfoxid wird mit 155 mg $K_2CO_3$ versetzt und unter Stickstoff 30 Minuten bei Raumtemperatur gerührt. Nach Zugabe von 0,2 ml 2-Chlorpyrazin wird die Mischung 6 Stunden bei 95 °C gerührt, dann in 1N Essigsäure gegossen und mit Ethylacetat extrahiert. Nach Aufreinigung über Kieselgel erhält man 242 mg (64 %), Schmelzpunkt 130-131 °C (Diethylether).

Das Ausgangsmaterial wird durch katalytische Hydrierung ($Pd/C/H_2$ in EtOH) des 5-Benzyloxy-4-methoxymethyl-β-carbolin-3-carbonsäure-ethylesters gewonnen.

Beispiel 9

4-Methoxymethyl-5-(2-pyrimidinyloxy)-β-carbolin-3-carbonsäure-ethylester

Analog Beispiel 8 aus 2-Chlorpyrimidin in Acetonitril, Schmelzpunkt 96-98 °C (EtOH).

Beispiel 10

5-(5-Chlor-2-pyridyloxy)-4-methoxymethyl-β-carbolin-3-carbonsäure-ethylester

Analog Beispiel 8 aus 2-Brom-5-chlorpyridin in Dimethylformamid, Schmelzpunkt 156-158 °C (Diisopropylether).

Beispiel 11

5-(5-Chlor-2-pyridiyloxy)-4-methyl-β-carbolin-3-carbonsäure-ethylester

Analog Beispiel 8 aus 5-Hydroxy-4-methyl-β-carbolin-2-carbonsäure-ethylester und 2-Brom-5-chlorpyridin in Dimethylformamid, Schmelzpunkt 194-196 °C (EtOH).

Beispiel 12

6-(5-Nitro-2-pyridyloxy)-β-carbolin-3-carbonsäure-methylester

Analog Beispiel 8 aus 6-Hydroxy-β-carbolin-3-carbonsäure-methylester und 2-Chlor-5-nitropyridin.

Schmelzpunkt 150 - 155 °C.

Beispiel 13

4-Methoxymethyl-6-(2-pyrimidinyloxy)-β-carbolin-3-carbonsäure-ethylester

Analog Beispiel 8 aus 6-Hydroxy-4-methoxymethyl-β-carbolin-3-carbonsäure-ethylester und 2-Chlorpyrimidin. Schmelzpunkt 128 - 129 °C.

Beispiel 14

6-(5-Brom-2-pyridyloxy)-4-methoxymethyl-β-carbolin-3-carbonsäure-ethylester

Analog Beispiel 8 aus 6-Hydroxy-4-methoxymethyl-β-carbolin-3-carbonsäure-ethylester mit 2,5-Dibrompyridin. Schmelzpunkt 210 -212 °C.

Beispiel 15

5-(2-Pyrimidinyloxy)-β-carbolin-3-carbonsäure-isopropylester

Eine Suspension von 185 mg 5-(2-Pyrimdinyloxy)-β-carbolin-3-carbonsäure-ethylester in 20 ml abs. 2-Propanol wird mit 0,16 ml Titanisopropylat versetzt und 90 Minuten unter Argonatmosphäre gekocht. Nach dem Einengen und Aufreinigen über Kieselgel erhält man 124 mg (64 %) Isopropylester, Schmelzpunkt 298-300 °C (Isopropanol).

Beispiel 16

5-(5-Chlor-2-pyridyloxy)-4-methoxymethyl-β-carbolin-3-carbonsäure-isopropylester

Analog Beispiel 15 aus dem entsprechenden Ethylester, Schmelzpunkt 190-191 °C (Isopropanol).

Beispiel 17

5-(5-Chlor-2-pyridyloxy)-4-methyl-β-carbolin-3-carbonsäure-isopropylester

Analog Beispiel 15 aus dem entsprechenden Ethylester, Schmelzpunkt 243-245 °C (Isopropanol).

Beispiel 18

6-(5-Brom-2-pyridyloxy)-4-methoxymethyl-β-carbolin-3-carbonsäure-isopropylester

Analog Beispiel 15 aus dem entsprechenden Ethylester, Schmelzpunkt 210-212 °C.

Beispiel 19

4-Methoxymethyl-6-(2-pyrimidinyloxy)-β-carbolin-3-carbonsäure-isopropylester

Analog Beispiel 15 aus dem entsprechenden Ethylester, Schmelzpunkt 166-169 °C.

Beispiel 20

5-(2-Pyrazinyloxy)-4-methoxymethyl-β-carbolin-3-carbonsäure

Eine Suspension von 325 mg 4-Methoxymethyl-5-(2-pyrazinyloxy)-β-carbolin-3-carbonsäure-ethylester in 2,5 ml 1N Natronlauge wird 30 Minuten auf 110 °C erhitzt. Nach Abkühlen wird mit 2N HCl auf pH 3 eingestellt, das Kristallisat abgesaugt und nachgewaschen. Man erhält 265 mg (88 %), Schmelzpunkt 236-237 °C.

In analoger Weise werden erhalten:

4-Methoxymethyl-5-(2-pyrimidinyloxy)-β-carbolin-3-carbonsäure, Schmelzpunkt 237-239 °C
5-(5-Chlor-2-pyridyloxy)-4-methoxymethyl-β-carbolin-3-carbonsäure, Schmelzpunkt 226-227 °C.

Beispiel 21

3-(3-Ethyl-1,2,4-oxadiazol-5-yl)-4-methoxymethyl-5-(2-pyrazinyloxy)-β-carbolin

Eine Lösung von 245 mg 4-Methoxymethyl-5-(2-pyrazinyloxy)-β-carbolin-3-carbonsäure in 15 ml Dimethylformamid wird mit 140 mg N, N'-Carbonyl-diimidazol versetzt und 1 Stunde bei 50°C gerührt. Dann werden 310 mg Propioamidoxim zugefügt und die Mischung 8 Stunden bei Raumtemperatur, anschließend noch 2 Stunden bei 100 °C gerührt. Nach Einengen im Vakuum wird der Rückstand mit 20 ml Xylol versetzt und 3 Stunden unter Rückfluß gekocht. Die filtrierte Xylolphase wird eingeengt und der Rückstand über Kieselgel aufgereinigt. Man erhält 170 mg (60 %); Schmelzpunkt192-193 °C (Ethanol).

Analog erhält man:
3-(3-Ethyl-1,2,4-oxadiazol-5-yl)-4-methoxymethyl-5-(2-pyrimidinyloxy)-β-carbolin, Schmelzpunkt 175-176 °C (EtOH)
5-(5-Chlor-2-pyridyloxy)-4-methoxymethyl-3-(3-ethyl-1,2,4-oxadiazol-5-yl)-β-carbolin, Schmelzpunkt 144-146 °C (Diisopropylether).

Beispiel 22

5-(5-Chlor-2-pyridyloxy)-4-methoxymethyl-β-carbolin-3-carbonsäure-isopropylamid

aus dem 3-Carbonsäure-imidazolid und Isopropylamin.

Beispiel 23

4-Methoxymethyl-5-(2-pyridyloxy)-β-carbolin-3-carbonsäure-isopropylester

Eine Suspension von 124 mg 5-(5-Chlor-2-pyridyloxy)-4-methoxymethyl-β-carbolin-3-carbonsäure-isopropylester in 15 ml 2-Propanol wird mit 50 mg Triethylamin und 120 mg Pd-C (10 %) versetzt und bei Raumtemperatur und Normaldruck hydriert. Nach Aufnahme der berechneten Menge an Wasserstoff wird filtriert, das Filtrat eingeengt und der Rückstand aus 2-Propanol umkristallisiert. Man erhält 96 mg (84 %), Schmelzpunkt 188-189 °C.

Beispiel 24

6-(5-Amino-2-pyridyloxy)-β-carbolin-3-carbonsäure-methylester

Eine Suspension von 3,65 g 6-(5-Nitro-2-pyridyloxy)-β-carbolin-3-carbonsäure-methylester und 0,5 g Pd/C (10 %) in 100 ml Methanol wird bei Raumtemperatur und Normaldruck hydriert. Nach Aufnahme der berechneten Menge Wasserstoff wird filtriert und eingeengt. Der Rückstand wird aus Methanol/Diethylether kristallisiert. Man erhält 2,84 g (85 %).

Beispiel 25

6-(5-Cyano-2-pyridyloxy)-β-carbolin-3-carbonsäure-methylester

Eine Suspension von 1,7 g Aminoderivat (Beispiel 24) in 10 ml Wasser und 2,5 ml Salzsäure (37 %) wird bei - 5°C mit einer Lösung von 0,4 g $NaNO_2$ in 1,5 ml Wasser tropfenweise versetzt, anschließend noch 1 Stunde bei 0 - 5 °C gerührt. Durch Zugabe von Natriumcarbonat wird dann die Lösung auf pH 5,5 - 6 eingestellt und in eine auf ca. 60°C vorgeheizte Mischung aus 0,5 g Kupfer-I-cyanid und 1,6 g Kaliumcyanid in 10 ml Wasser eingegossen. Nach Beendigung der Reaktion wird die abgekühlte Lösung mit Dichlormethan extrahiert, die organische Phase mit Wasser gewaschen und eingeengt. Der Rückstand wird über Kieselgel aufgereinigt. Man erhält 1,12 g (65 %).

Beispiel 26

4-Methoxymethyl-5-(5-nitro-2-thiazolyloxy)-β-carbolin-3-carbonsäure ethylester

Analog Beispiel 8 aus 2-Brom-5-nitro-thiazol.

Beispiel 27

5-(5-Ethoxycarbonyl-2-furyloxy)-4-methoxymethyl-β-carbolin-3-carbonsäure-ethylester

Analog Beispiel 8 aus 5-Brom-furan-2-carbonsäure-ethylester.

Beispiel 28

5-(5-Formyl-2-thienyloxy)-4-methoxymethyl-β-carbolin-3-carbonsäure-ethylester

Analog Beispiel 8 aus 5-Brom-thiophen-2-carbaldehyd.

Beispiel 29

4-Methoxymethyl-5-(5-nitro-2-thiazolyloxy)-β-carbolin-3-carbonsäure-isopropylester

Dargestellt analog Beispiel 8 aus 5-Hydroxy-4-methoxymethyl-β-carbolin-3-carbonsäure-ethylester und 2-Brom-5-nitro-thiazol und anschließende Umesterung analog Beispiel 15. Schmelzpunkt 190-2 ° C (i-PrOH).

Beispiel 30

5-(5-Ethoxycarbonyl-2-furyloxy)-4-methoxymethyl-β-carbolin-3-carbonsäure-isopropylester

Dargestellt analog Beispiel 29 aus 5-Brom-furan-2-carbonsäure-ethylester. Schmelzpunkt 191-2 ° C (i-PrOH).

Beispiel 31

4-Methoxymethyl-5-(2-thiazolyloxy)-β-carbolin-3-carbonsäure-isopropylester

Dargestellt analog Beispiel 29 aus 2-Brom-thiazol. Schmelzpunkt 123-125 ° C (Ethylacetat).

Beispiel 32

6-(5-Brom-2-pyridyloxy)-4-methoxymethyl-β-carbolin-3-carbonsäure-tert. butylester

Dargestellt aus der entsprechenden Säure durch Erhitzen mit tert. Butoxy- bis (dimethylamino)-methan. Schmelzpunkt 173-75 ° C.

**Patentansprüche**

**1.** Hetaryloxy-β-carbolinderivate der allgemeinen Formel I

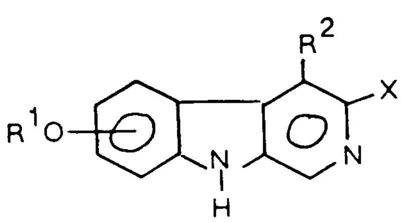

(I),

worin

$R^1$ ein 6-Ring-Hetarylrest mit 1-2 Stickstoffatomen oder ein 5-Ring-Hetarylrest mit 1-2 Sauerstoff-, Schwefel- und/oder Stickstoffatomen, der gegebenenfalls ein- oder mehrfach substituiert ist mit Halogenen, Nitro, Amino, Cyano, $C_{1-6}$-Alkyl oder $C_{1-6}$ Alkoxy-carbonyl.
$R^2$ Wasserstoff, $C_{1-6}$-Alkyl oder $C_{1-6}$-Alkoxyalkyl und
X eine $COOR^3$-Gruppe mit $R^3$ in der Bedeutung von H oder $C_{1-6}$-Alkyl oder
eine $CONR^4R^5$-Gruppe darstellt mit $R^4$ und $R^5$ jeweils in der Bedeutung von Wasserstoff oder $C_{1-3}$-Alkyl, wobei $R^4$ und $R^5$ gemeinsam mit dem Stickstoffatom Morpholin, Piperidin, Thiomorpholin, Piperazin, Pyrrolidin, Imidazolidin, Pyrazolidin, Isothiazolidin bilden können oder
einen Oxadiazolylrest der Formel

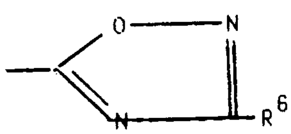
oder
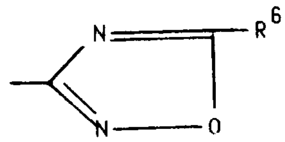

mit $R^6$ in der Bedeutung von Wasserstoff, $C_{1-6}$-Alkyl oder $C_{3-5}$-Cycloalkyl bedeutet.

**2.** 5-(2-Pyrazinyloxy)-β-carbolin-3-carbonsäure-ethylester
5-(5-Nitro-2-pyridyloxy)-β-carbolin-3-carbonsäure-ethylester
5-(2-Pyrimidinyloxy)-β-carbolin-3-carbonsäure-ethylester
3-(3-Ethyl-1,2,4-oxadiazol-5-yl)-5-(2-pyrazinyloxy)-β-carbolin
5-(5-Chlor-2-pyridyloxy)-3-(3-ethyl-1,2,4-oxadiazol-5-yl)-β-carbolin
5-(2-Pyrimidinyloxy)-3-(3-ethyl-1,2,4-oxadiazol-5-yl)-β-carbolin
5-(5-Nitro-2-pyridyloxy)-3-(3-ethyl-1,2,4-oxadiazol-5-yl)-β-carbolin
4-Methoxymethyl-5-(2-pyrazinyloxy)-β-carbolin-3-carbonsäure-ethylester
4-Methoxymethyl-5-(2-pyrimidinyloxy)-β-carbolin-3-carbonsäure-ethylester
5-(5-Chlor-2-pyridyloxy)-4-methoxymethyl-β-carbolin-3-carbonsäure-ethylester
5-(5-Chlor-2-pyridyloxy)-4-methyl-β-carbolin-3-carbonsäure-ethylester
6-(5-Nitro-2-pyridyloxy)-β-carbolin-3-carbonsäure-methylester
4-Methyloxymethyl-6-(2-pyrimidinyloxy)-β-carbolin-3-carbonsäure-ethylester
6-(5-Brom-2-pyridyloxy)-4-methoxymethyl-β-carbolin-3-carbonsäure-ethylester
5-(2-Pyrimidinyloxy)-β-carbolin-3-carbonsäure-isopropylester
5-(5-Chlor-2-pyridyloxy)-4-methoxymethyl-β-carbolin-3-carbonsäure-isopropylester
5-(5-Chlor-2-pyridyloxy)-4-methyl-β-carbolin-3-carbonsäure-isopropylester
6-(5-Brom-2-pyridyloxy)-4-methoxymethyl-β-carbolin-3-carbonsäure-isopropylester
4-Methoxymethyl-6-(2-pyrimidinyloxy)-β-carbolin-3-carbonsäure-isopropylester
5-(2-Pyrazinyloxy)-4-methoxymethyl-β-carbolin-3-carbonsäure
4-Methoxymethyl-5-(2-pyrimidinyloxy)-β-carbolin-3-carbonsäure
5-(5-Chlor-2-pyridinyloxy)-4-methoxymethyl-β-carbolin-3-carbonsäure
3-(3-Ethyl-1,2,4-oxadiazol-5-yl)-4-methoxymethyl-5-(2-pyrazinyloxy)-β-carbolin
3-(3-Ethyl-1,2,4-oxadiazol-5-yl)-4-methoxymethyl-5-(2-pyrimidinyloxy)-β-carbolin
5-(5-Chlor-2-pyridinyl)-4-methoxymethyl-3-(3-ethyl-1,2,4-oxadiazol-5-yl)-β-carbolin
5-(5-Chlor-2-pyridinyloxy)-4-methoxymethyl-β-carbolin-3-carbonsäure-isopropylamid
4-Methoxymethyl-5-(2-pyridyloxy)-β-carbolin-3-carbonsäure-isopropylester
6-(5-Amin o-2-pyridyloxy)-β-carbolin-3-carbonsäure-methylester
6-(5-Cyano-2-pyridyloxy)-β-carbolin-3-carbonsäure-methylester
4-Methoxymethyl-5-(5-nitro-2-thiazolyloxy)-β-carbolin-3-carbonsäure-ethylester
5-(5-Ethoxycarbonyl-2-furyloxy)-4-methoxymethyl-β-carbolin-3-carbonsäure-ethylester
5-(5-Formyl-2-thienyloxy)-4-methoxymethyl-β-carbolin-3-carbonsäure-ethylester nach Anspruch 1.

**3.** Verfahren zur Herstellung der Verbindung der allgemeinen Formel I dadurch, daß man
a) ein Indol der allgemeinen Formel II

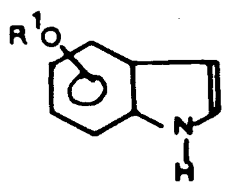

(II),

worin $R^1$ die in Formel I angegebene Bedeutung hat, mit einem Azabutadien der Formel III

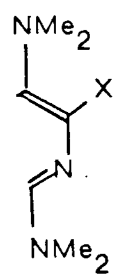

(III),

worin X eine $COOR^3$-Gruppe mit $R^3$ in der Bedeutung von $C_{1-6}$-Alkyl oder einen Oxadiazolylrest der Formel

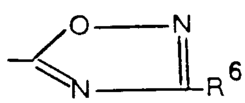

oder

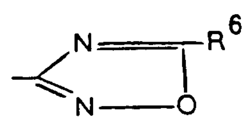

mit $R^6$ in der oben genannten Bedeutung darstellt, in Gegenwart von Säuren umsetzt oder
b) ein $\beta$-Carbolinderivat der allgemeinen Formel IV

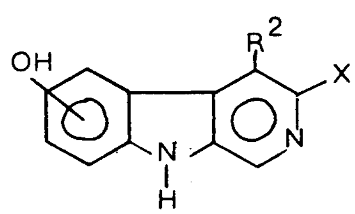

(IV),

worin X und $R^2$ die oben angegebene Bedeutung haben, mit Halogen $R^1$ veräthert, wobei $R^1$ die oben angegebene Bedeutung hat oder
c) eine Verbindung der Formel V

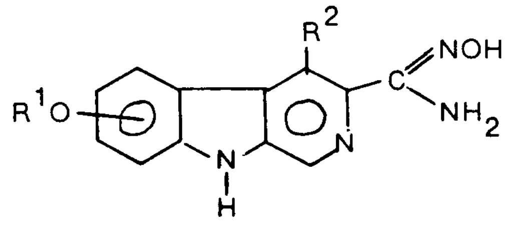

(V),

worin $R^1$ und $R^2$ die oben angegebene Bedeutung haben mit einer Verbindung der Formel $(R^6CO)_2O$ mit $R^6$ in der oben genannten Bedeutung umsetzt zu einer Verbindung der allgemeinen Formel I mit

X in der Bedeutung von

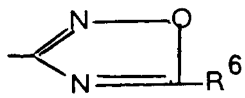

mit $R^6$ in der oben genannten Bedeutung und anschließend gegebenenfalls die nach Verfahren a), b) oder c) hergestellten Verbindungen

α) mit $R^1$ in der Bedeutung von $O_2N$-Hetaryl reduziert zu $H_2N$-Hetaryl-Verbindungen und diese gewünschtenfalls anschließend in N≡C-Hetaryl-Verbindungen überführt und

β) mit $R^1$ in der Bedeutung von Halogenhetaryl enthalogeniert und

γ) mit X in der Bedeutung von $COOR^3$, worin $R^3$ $C_{1-6}$-Alkyl bedeutet, umestert oder verseift und die so erhaltenen Verbindungen mit $R^3$ in der Bedeutung von Wasserstoff gewünschtenfalls amidiert zu Verbindungen mit X in der Bedeutung von $CONR^4R^5$, wobei $R^4$ und $R^5$ die oben genannte Bedeutung haben oder mit einem Amidoxim der Formel $R^6$-$CNH_2$(=NOH), worin $R^6$ die oben angegebene Bedeutung hat, umsetzt zu Verbindungen mit X in der Bedeutung von

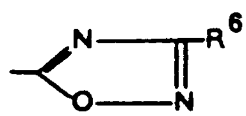

worin $R^6$ die oben angegebene Bedeutung hat.

**4.** Verwendung der Verbindungen nach den Ansprüchen 1 und 2 zur Herstellung eines Arzneimittels.

**5.** Arzneimittel auf Basis der Verbindungen nach den Ansprüchen 1 und 2.

**6.** Verfahren zur Herstellung eines Arzneimittels, dadurch gekennzeichnet, daß eine Verbindung der allgemeinen Formel I nach Anspruch 1 durch Mischen mit pharmazeutisch annehmbaren Träger- und/oder Zusatzstoffen in eine zur Verabreichung geeignete Form gebracht wird.

## Claims

**1.** Heteroaryloxy-β-carboline derivatives of the general formula I

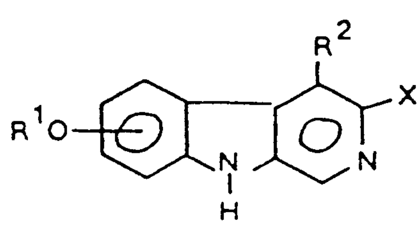

(I)

wherein

$R^1$ represents a 6-membered ring heteroaryl radical having 1 or 2 nitrogen atoms or a 5-membered ring heteroaryl radical having 1 or 2 oxygen, sulphur and/or nitrogen atoms, the radical optionally being mono- or poly-substituted by halogens, nitro, amino, cyano, $C_{1-6}$-alkyl or by $C_{1-6}$-alkoxycarbonyl;

$R^2$ represents hydrogen, $C_{1-6}$-alkyl or $C_{1-6}$-alkoxyalkyl; and

X represents a $COOR^3$ group in which $R^3$ is H or $C_{1-6}$-alkyl, or represents a $CONR^4R^5$ group in which each of $R^4$ and $R^5$ is hydrogen or $C_{1-3}$-alkyl, it being possible for $R^4$ and $R^5$, together with the nitrogen atom, to form morpholine, piperidine, thiomorpholine, piperazine, pyrrolidine, imidazolidine, pyrazolidine

or isothiazolidine, or X represents an oxadiazolyl radical of the formula

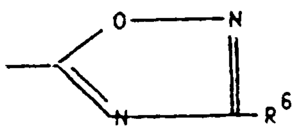 or 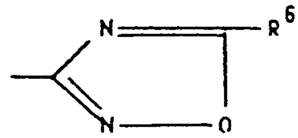

in which $R^6$ is hydrogen, $C_{1-6}$-alkyl or $C_{3-5}$-cycloalkyl.

**2.** 5-(2-pyrazinyloxy)-β-carboline-3-carboxylic acid ethyl ester,
5-(5-nitro-2-pyridyloxy)-β-carboline-3-carboxylic acid ethyl ester,
5-(2-pyrimidinyloxy)-β-carboline-3-carboxylic acid ethyl ester,
3-(3-ethyl-1,2,4-oxadiazol-5-yl)-5-(2-pyrazinyloxy)-β-carboline,
5-(5-chloro-2-pyridyloxy)-3-(3-ethyl-1,2,4-oxadiazol-5-yl)-β-carboline,
5-(2-pyrimidinyloxy)-3-(3-ethyl-1,2,4-oxadiazol-5-yl)-β-carboline,
5-(5-nitro-2-pyridyloxy)-3-(3-ethyl-1,2,4-oxadiazol-5-yl)-β-carboline,
4-methoxymethyl-5-(2-pyrazinyloxy)-β-carboline-3-carboxylic acid ethyl ester,
4-methoxymethyl-5-(2-pyrimidinyloxy)-β-carboline-3-carboxylic acid ethyl ester,
5-(5-chloro-2-pyridyloxy)-4-methoxymethyl-β-carboline-3-carboxylic acid ethyl ester,
5-(5-chloro-2-pyridyloxy)-4-methyl-β-carboline-3-carboxylic acid ethyl ester,
6-(5-nitro-2-pyridyloxy)-β-carboline-3-carboxylic acid methyl ester,
4-methoxymethyl-6-(2-pyrimidinyloxy)-β-carboline-3-carboxylic acid ethyl ester,
6-(5-bromo-2-pyridyloxy)-4-methoxymethyl-β-carboline-3-carboxylic acid ethyl ester,
5-(2-pyrimidinyloxy)-β-carboline-3-carboxylic acid isopropyl ester,
5-(5-chloro-2-pyridyloxy)-4-methoxymethyl-β-carboline-3-carboxylic acid isopropyl ester,
5-(5-chloro-2-pyridyloxy)-4-methyl-β-carboline-3-carboxylic acid isopropyl ester,
6-(5-bromo-2-pyridyloxy)-4-methoxymethyl-β-carboline-3-carboxylic acid isopropyl ester,
4-methoxymethyl-6-(2-pyrimidinyloxy)-β-carboline-3-carboxylic acid isopropyl ester,
5-(2-pyrazinyloxy)-4-methoxymethyl-β-carboline-3-carboxylic acid,
4-methoxymethyl-5-(2-pyrimidinyloxy)-β-carboline-3-carboxylic acid,
5-(5-chloro-2-pyridyloxy)-4-methoxymethyl-β-carboline-3-carboxylic acid,
3-(3-ethyl-1,2,4-oxadiazol-5-yl)-4-methoxymethyl-5-(2-pyrazinyloxy)-β-carboline,
3-(3-ethyl-1,2,4-oxadiazol-5-yl)-4-methoxymethyl-5-(2-pyrimidinyloxy)-β-carboline,
5-(5-chloro-2-pyridyloxy)-4-methoxymethyl-3-(3-ethyl-1,2,4-oxadiazol-5-yl)-β-carboline,
5-(5-chloro-2-pyridyloxy)-4-methoxymethyl-β-carboline-3-carboxylic acid isopropylamide,
4-methoxymethyl-5-(2-pyridyloxy)-β-carboline-3-carboxylicacid isopropyl ester,
6-(5-amino-2-pyridyloxy)-β-carboline-3-carboxylic acid methyl ester,
6-(5-cyano-2-pyridyloxy)-β-carboline-3-carboxylic acid methyl ester,
4-methoxymethyl-5-(5-nitro-2-thiazolyloxy)-β-carboline-3-carboxylic acid ethyl ester,
5-(5-ethoxycarbonyl-2-furyloxy)-4-methoxymethyl-β-carboline-3-carboxylic acid ethyl ester,
5-(5-formyl-2-thienyloxy)-4-methoxymethyl-β-carboline-3-carboxylic acid ethyl ester,
according to claim 1.

**3.** Process for the preparation of the compounds of the general formula I, characterised in that
a) an indole of the general formula II

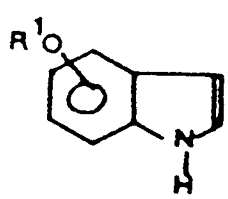 (II),

wherein $R^1$ has the meaning given in formula I, is reacted, in the presence of acids, with an azabutadiene of formula III

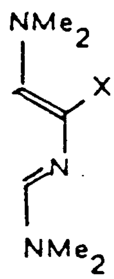

(III),

wherein X represents a $COOR^3$ group in which $R^3$ is $C_{1-6}$-alkyl, or represents an oxadiazolyl radical of the formula

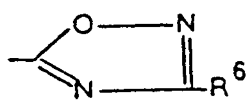

or

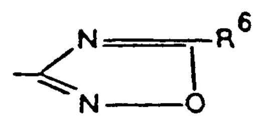

in which $R^6$ has the meaning given above, or
b) a *β*-carboline derivative of the general formula IV

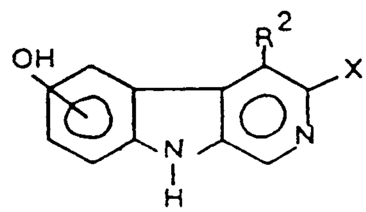

(IV),

wherein X and $R^2$ have the meaning given above, is etherified by means of halogen-$R^1$, $R^1$ having the meaning given above, or
c) a compound of formula V

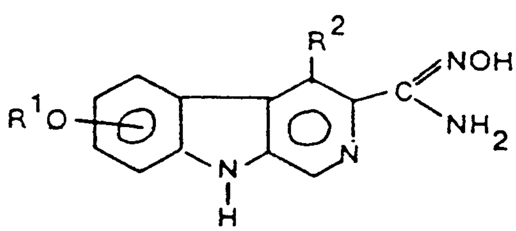

(V),

wherein $R^1$ and $R^2$ have the meaning given above, is reacted with a compound of the formula $(R^6CO)_2O$, in which $R^6$ has the meaning given above, to form a compound of the general formula I wherein X represents

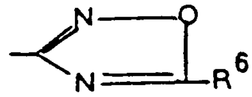

,

in which $R^6$ has the meaning given above,
and then, optionally, the compounds prepared according to process a), b) or c)

α) wherein $R^1$ represents $O_2N$-heteroaryl, are reduced to form $H_2N$-heteroaryl compounds, which, if desired, are then converted into N≡C-heteroaryl compounds, and

β) wherein $R^1$ represents haloheteroaryl, are dehalogenated, and
γ) wherein X represents $COOR^3$, in which $R^3$ is $C_{1-6}$-alkyl, are transesterified or hydrolysed, and the resulting compounds wherein $R^3$ represents hydrogen are, if desired, amidated to form compounds wherein X represents $CONR^4R^5$, in which $R^4$ and $R^5$ have the meaning given above, or are reacted with an amidoxime of the formula $R^6$-$CNH_2(=NOH)$,in which $R^6$ has the meaning given above, to form compounds wherein X represents

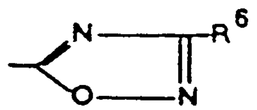 ,

in which $R^6$ has the meaning given above.

**4.** Use of the compounds according to claims 1 and 2 for the preparation of a medicament.

**5.** Medicament based on the compounds according to claims 1 and 2.

**6.** Process for preparing a medicament characterized in that a compound of the general formula I described in claim 1 is converted into a form suitable for administration by mixing with pharmacologically acceptable excipients and/or additives.

## Revendications

**1.** Hétaryloxy-β-carbolines répondent à la formule générale I :

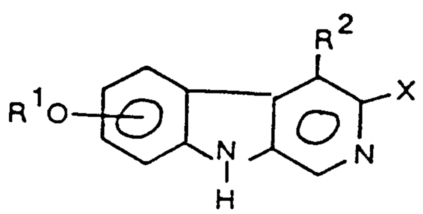 (I),

dans laquelle

$R^1$ représente un radical hétaryle hexagonal contenant 1 ou 2 atomes d'azote ou un radical hétaryle pentagonal contenant 1 ou 2 atomes d'oxygène, de soufre et/ou d'azote, le radical hétaryle en question portant éventuellement un ou plusieurs substituants pris dans l'ensemble constitué par les halogènes et les radicaux nitro, amino, cyano, alkyles en $C_1$-$C_6$ et ($C_1$-$C_6$-alcoxy)-carbonyles,

$R^2$ représente l'hydrogène, un alkyle en $C_1$-$C_6$ ou un ($C_1$-$C_6$-alcoxy)-alkyle et

X représente :

un radical $COOR^3$ dans lequel $R^3$ représente H ou un alkyle en $C_1$-$C_6$,

un radical $CONR^4R^5$ dans lequel $R^4$ et $R^5$ représentent chacun l'hydrogène ou un alkyle en $C_1$-$C_3$ ou peuvent former ensemble, et avec l'atome d'azote, un radical de morpholine, de pipéridine, de thiomorpholine, de pipérazine, de pyrrolidine, d'imidazolidine, de pyrazolidine ou d'isothiazolidine, ou

un radical oxadiazolyle répondant à l'une ou l'autre des formules :

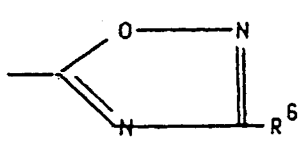 et 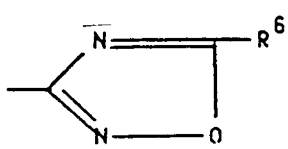

dans lesquelles $R^6$ représente l'hydrogène, un alkyle en $C_1$-$C_6$ ou un cycloalkyle en $C_3$-$C_5$.

**2.** Composés selon la revendication 1, en l'espèce :
le 5-(2-pyrazinyloxy)-β-carboline-3-carboxylate d'éthyle
le 5-(5-nitro-2-pyridyloxy)-β-carboline-3-carboxylate d'éthyle
le 5-(2-pyrimidinyloxy)-β-carboline-3-carboxylate-d'éthyle
la 3-(3-éthyl-1,2,4-oxadiazole-5-yl)-5-(2-pyrazinyloxy)-β-carboline
la 5-(5-chloro-2-pyridyloxy)-3-(3-éthyl-1,2,4-oxadiazole-5-yl)-β-carboline
la 5-(2-pyrimidinyloxy)-3-(3-éthyl-1,2,4-oxadiazole-5-yl)-β-carboline
la 5-(5-nitro-2-pyridyloxy)-3-(3-éthyl-1,2,4-oxadiazole-5-yl)-β-carboline
le 4-méthoxyméthyl-5-(2-pyrazinyloxy)-β-carboline-carboxylate d'éthyle
le 4-méthoxyméthyl-5-(2-pyrimidinyloxy)-β-carboline-3-carboxylate d'éthyle
le 5-(5-chloro-2-pyridyloxy)-4-méthoxyméthyl-β-carboline-carboxylate d'éthyle
le 5-(5-chloro-2-pyridyloxy)-4-méthyl-β-carboline-3-carboxylate d'éthyle
le 6-(5-nitro-2-pyridyloxy)-β-carboline-3-carboxylate de méthyle
le 4-méthoxyméthyl-6-(2-pyrimidinyloxy)-β-carboline-3-carboxylate d'éthyle
le 6-(5-bromo-2-pyridyloxy-4-méthoxyméthyl-β-carboline-3-carboxylate d'éthyle
le 5-(2-pyrimidinyloxy)-β-carboline-3-carboxylate-3 d'isopropyle
le 5-(5-chloro-2-pyridyloxy)-4méthoxyméthyl-β-carboline-3-carboxylate d'isopropyle
le 5-(5-chloro-2-pyridyloxy)-4-méthyl-β-carboline-carboxylate-3 d'isopropyle
le 6-(5-bromo-2-pyridyloxy)-4-méthoxyméthyl-β-carboline-3-carboxylate d'isopropyle
le 4-méthoxyméthyl-6-(2-pyrimidinyloxy)-β-carboline-3-carboxylate d'isopropyle
l'acide 5-(2-pyrazinyloxy)-4-méthoxyméthyl-β-carboline-3-carboxylique
l'acide 4-méthoxyméthyl-5-(2-pyrimidinyloxy)-β-carboline-3-carboxylique
l'acide 5-(5-chloro-2-pyridyloxy)-4-méthoxyméthyl-β-carboline-3-carboxylique,
la 3-(3-éthyl-1,2,4-oxadiazole-5-yl)-4-méthoxyméthyl-5-(2-pyrazinyloxy)-β-carboline
la 3-(3-éthyl-1,2,4-oxadiazol-5-yl)-4-méthoxyméthyl-5-(2-pyrimidinyloxy)-β-carboline
la 5-(5-chloro-2-pyridyloxy)-4-méthoxyméthyl-3-(3-éthyl-1,2,4-oxadiazole-5-yl)-β-carboline
l'isopropylamide de l'acide 5-(5-chloro-2-pyridyloxy)-4-méthoxyméthy-β-carboline-3-carboxylique
le 4-méthoxyméthyl-5-(2-pyridyloxv)-β-carboline-3-carboxylate d'isopropyle
le 6-(5-amino-2-pyridyloxy)-β-carboline-3-carboxylate de méthyle
le 6-(5-cyano-2-pyridyloxy)-β-carboline-3-carboxylate de méthyle
le 4-méthoxyméthyl-5-(5-nitro-2-thiazolyloxy)-β-carboline-3-carboxylate d'éthyle
le 5-(5-éthoxycarbonyl-2-furyloxy)-4-méthoxyméthyl-β-carboline-carboxylate-3 d'éthyle et
le 5-(5-formyl-2-thiényloxy)-4-méthoxyméthyl-β-carboline-3-carboxylate d'éthyle.

**3.** Procédé pour préparer les composés de formule générale I, procédé caractérisé en ce que :
a) on fait réagir, en présence d'acides, un indole répondant à la formule générale II :

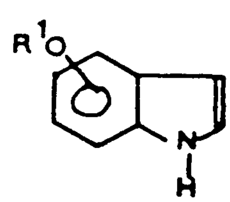

(II),

dans laquelle $R^1$ a la signification qui lui a été donnée à propos de la formule I, avec un azabutadiène répondant à la formule III :

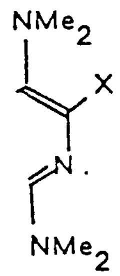

(III),

dans laquelle X représente un radical -COOR$^3$ (dont le symbole R$^3$ désigne un alkyle en $C_1$-$C_6$) ou un radical oxadiazolyle répondant à l'une des formules :

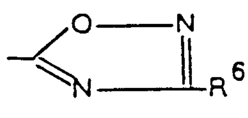

et

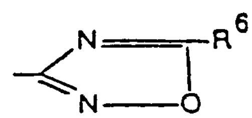

dans lesquelles R$^6$ a la signification indiquée plus haut, ou
b) on éthérifie une $\beta$-carboline répondant à la formule générale IV :

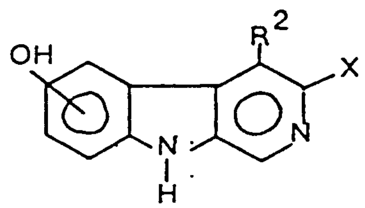

(IV),

dans laquelle X et R$^2$ ont les significations indiquées ci-dessus, avec un composé halogéno-R$^1$ dans lequel R$^1$ a la signification indiquée ci-dessus, ou
c) on fait réagir un composé répondant à la formule V :

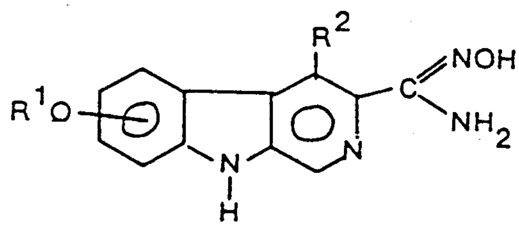

(V),

dans laquelle R$^1$ et R$^2$ ont les significations indiquées ci-dessus, avec un composé de formule $(R^6CO)_2O$ dans lequel R$^6$ a la signification indiquée ci-dessus, de manière à obtenir un composé de formule générale I dans lequel X représente un radical :

dans lequel $R^6$ a la signification indiquée ci-dessus, puis, le cas échéant, on effectue sur les composés préparés par la méthode a), b) ou c) les réactions suivantes :

α) lorsque $R^1$ représente un radical $O_2N$-hétaryle on les réduit en composés $H_2N$-hétaryliques et, si on le désire, on transforme ensuite ceux-ci en composés N = C-hétaryliques,

β) lorsque $R^1$ représente un radical halogéno-hétaryle on les déshalogène, et

γ) lorsque X représente un radical $-COOR^3$ dans lequel $R^3$ représente un alkyle en $C_1$-$C_6$ on les estérifie ou les saponifie, et, si on le désire, on amide les composés ainsi obtenus dans lesquels $R^3$ représente l'hydrogène, de manière à obtenir des composés dans lesquels X représente un radical $-CONR^4R^5$ (dans lequel $R^4$ et $R^5$ ont les significations indiquées ci-dessus) ou on les fait réagir avec une amide-oxime de formule $R^6-CNH_2(=NOH)$ dans laquelle $R^6$ a la signification indiquée ci-dessus, de manière à obtenir des composés dans lesquels X représente un radical :

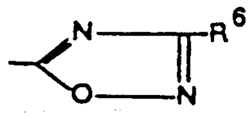

dans lequel $R^6$ a la signification indiquée ci-dessus.

**4.** Application des composés selon l'une des revendications 1 et 2 à la préparation d'un médicament.

**5.** Médicaments à base des composés selon l'une des revendications 1 et 2.

**6.** Procédé de confection d'un médicament caracterisé en ce qu'on amène sous une forme d'administration appropriée une composé à la formule générale I indiquée dans la revendication 1 par mélange avec des excipients et/ou additifs pharmacologiquement acceptables.